# EUROPEAN PATENT SPECIFICATION

(11) **EP 2 670 436 B1**
(45) Date of publication and mention of the grant of the patent: **12.07.2017**
(21) Application number: 12741931.5
(22) Date of filing: 03.02.2012
(51) Int. Cl.: A61K 39/395, C07K 16/18

(54) **FOXC1 ANTIBODIES AND METHODS OF THEIR USE**
FOXC1-ANTIKÖRPER UND VERFAHREN ZU IHRER VERWENDUNG
ANTICORPS ANTI-FOXC1 ET LEURS PROCÉDÉS D'UTILISATION

(30) Priority: 04.02.2011 US 201161439825 P
(43) Date of publication of application: 11.12.2013
(73) Proprietor: John Wayne Cancer Institute, Santa Monica, CA 90404 (US)
(72) Inventor: CUI, Xiaojiang, Los Angeles California 90048 (US)
(74) Representative: Viering, Jentschura & Partner mbB Patent- und Rechtsanwälte
(86) International application number: PCT/US2012/023871
(87) International publication number: WO 2012/106669

(56) References cited:
- WO-A2-2009/009739
- US-A1- 2010 166 745
- RAY P S ET AL: "Role of FOXC1 in regulation of basal-like/triple-negative breast cancer", JOURNAL OF CLINICAL ONCOLOGY - (PROC OF ASCO VOLUME 19), , vol. 27, no. 15S 20 May 2009 (2009-05-20), page ABS11016, XP008159002, Retrieved from the Internet: URL:http://meeting.ascopubs.org/cgi/conten t/abstract/27/15S/11016
- JOSEPH H TAUBE ET AL: "Core epithelial-to-mesenchymal transition interactome gene-expression signature is associated with claudin-low and metaplastic breast cancer subtypes", NATIONAL ACADEMY OF SCIENCES. PROCEEDINGS, NATIONAL ACADEMY OF SCIENCES, UNITED STATES, vol. 107, no. 35, 31 August 2010 (2010-08-31), pages 15449-15454, XP002665255, ISSN: 1091-6490, DOI: 10.1073/PNAS.1004900107 [retrieved on 2010-08-16]
- MUGGERUD ASLAUG AA ET AL: "Frequent aberrant DNA methylation of ABCB1, FOXC1, PPP2R2B and PTEN in ductal carcinoma in situ and early invasive breast cancer", BREAST CANCER RESEARCH, CURRENT SCIENCE, LONDON, GB, vol. 12, no. 1, 7 January 2010 (2010-01-07), page R3, XP021070767, ISSN: 1465-5411
- BERRY ET AL.: 'FOXC1 Transcriptional Regulation Is Mediated by N- and C-terminal Activation Domains and Contains a Phosphorylated Transcriptional Inhibitory Domain' THE JOURNAL OF BIOLOGICAL CHEMISTRY vol. 277, no. 12, 22 March 2002, pages 10292 - 10297, XP055127059
- RAY ET AL.: 'FOXC1 Is a Potential Prognostic Biomarker with Functional Significance in Basal- like Breast Cancer.' CANCER RESEARCH. vol. 70, 20 April 2010, pages 3870 - 3876, XP008159009

### SEQUENCE LISTING

<110> JOHN WAYNE CANCER INSTITUTE
   Cui, Xiaojiang
<120> FOXC1 ANTIBODIES AND METHODS OF THEIR USE
<130> 79877.8002.WO00
<150> 61/439,825
   <151> 2011-02-04
<160> 3
<170> PatentIn version 3.5
<210> 1
   <211> 553
   <212> PRT
   <213> Homo sapiens
<300>
   <308> GenBank/AAH70124
   <309> 2006-07-15
   <313> (1)..(553)
<400> 1
   Met Gln Ala Arg Tyr Ser Val Ser Ser Pro Asn Ser Leu Gly Val Val
   1               5                   10                  15
   Pro Tyr Leu Gly Gly Glu Gln Ser Tyr Tyr Arg Ala Ala Ala Ala Ala
               20                  25                  30
   Ala Gly Gly Gly Tyr Thr Ala Met Pro Ala Pro Met Ser Val Tyr Ser
           35                  40                  45
   His Pro Ala His Ala Glu Gln Tyr Pro Gly Gly Met Ala Arg Ala Tyr
       50                  55                  60
   Gly Pro Tyr Thr Pro Gln Pro Gln Pro Lys Asp Met Val Lys Pro Pro
   65                  70                  75                  80
   Tyr Ser Tyr Ile Ala Leu Ile Thr Met Ala Ile Gln Asn Ala Pro Asp
                   85                  90                  95
   Lys Lys Ile Thr Leu Asn Gly Ile Tyr Gln Phe Ile Met Asp Arg Phe
               100                 105                 110
   Pro Phe Tyr Arg Asp Asn Lys Gln Gly Trp Gln Asn Ser Ile Arg His
           115                 120                 125
   Asn Leu Ser Leu Asn Glu Cys Phe Val Lys Val Pro Arg Asp Asp Lys
       130                 135                 140
   Lys Pro Gly Lys Gly Ser Tyr Trp Thr Leu Asp Pro Asp Ser Tyr Asn
   145                 150                 155                 160
   Met Phe Glu Asn Gly Ser Phe Leu Arg Arg Arg Arg Arg Phe Lys Lys
                   165                 170                 175
   Lys Asp Ala Val Lys Asp Lys Glu Glu Lys Asp Arg Leu His Leu Lys
               180                 185                 190
   Glu Pro Pro Pro Pro Gly Arg Gln Pro Pro Pro Ala Pro Pro Glu Gln
           195                 200                 205
   Ala Asp Gly Asn Ala Pro Gly Pro Gln Pro Pro Pro Val Arg Ile Gln
       210                 215                 220
   Asp Ile Lys Thr Glu Asn Gly Thr Cys Pro Ser Pro Pro Gln Pro Leu
   225                 230                 235                 240
   Ser Pro Ala Ala Ala Leu Gly Ser Gly Ser Ala Ala Ala Val Pro Lys
                   245                 250                 255
   Ile Glu Ser Pro Asp Ser Ser Ser Ser Ser Leu Ser Ser Gly Ser Ser
               260                 265                 270
   Pro Pro Gly Ser Leu Pro Ser Ala Arg Pro Leu Ser Leu Asp Gly Ala
           275                 280                 285
   Asp Ser Ala Pro Pro Pro Pro Ala Pro Ser Ala Pro Pro Pro His His
       290                 295                 300
   Ser Gln Gly Phe Ser Val Asp Asn Ile Met Thr Ser Leu Arg Gly Ser
   305                 310                 315                 320
   Pro Gln Ser Ala Ala Ala Glu Leu Ser Ser Gly Leu Leu Ala Ser Ala
                   325                 330                 335
   Ala Ala Ser Ser Arg Ala Gly Ile Ala Pro Pro Leu Ala Leu Gly Ala
               340                 345                 350
   Tyr Ser Pro Gly Gln Ser Ser Leu Tyr Ser Ser Pro Cys Ser Gln Thr
           355                 360                 365
   Ser Ser Ala Gly Ser Ser Gly Gly Gly Gly Gly Gly Ala Gly Ala Ala
       370                 375                 380
   Gly Gly Ala Gly Gly Ala Gly Thr Tyr His Cys Asn Leu Gln Ala Met
   385                 390                 395                 400
   Ser Leu Tyr Ala Ala Gly Glu Arg Gly Gly His Leu Gln Gly Ala Pro
                   405                 410                 415
   Gly Gly Ala Gly Gly Ser Ala Val Asp Asn Pro Leu Pro Asp Tyr Ser
               420                 425                 430
   Leu Pro Pro Val Thr Ser Ser Ser Ser Ser Ser Leu Ser His Gly Gly
           435                 440                 445
   Gly Gly Gly Gly Gly Gly Gly Gly Gln Glu Ala Gly His His Pro Ala
       450                 455                 460
   Ala His Gln Gly Arg Leu Thr Ser Trp Tyr Leu Asn Gln Ala Gly Gly
   465                 470                 475                 480
   Asp Leu Gly His Leu Ala Ser Ala Ala Ala Ala Ala Ala Ala Ala Gly
                   485                 490                 495
   Tyr Pro Gly Gln Gln Gln Asn Phe His Ser Val Arg Glu Met Phe Glu
               500                 505                 510
   Ser Gln Arg Ile Gly Leu Asn Asn Ser Pro Val Asn Gly Asn Ser Ser
           515                 520                 525
   Cys Gln Met Ala Phe Pro Ser Ser Gln Ser Leu Tyr Arg Thr Ser Gly
       530                 535                 540
   Ala Phe Val Tyr Asp Cys Ser Lys Phe
   545                 550
<210> 2
   <211> 25
   <212> PRT
   <213> Homo sapiens
<400> 2
   Ala His Ala Glu Gln Tyr Pro Gly Gly Met Ala Arg Ala Tyr Gly Pro
   1               5                   10                  15
   Tyr Thr Pro Gln Pro Gln Pro Lys Asp
               20                  25
<210> 3
   <211> 25
   <212> PRT
   <213> Homo sapiens
<400> 3
   Ala His Ala Glu Gln Tyr Pro Gly Gly Met Ala Arg Ala Tyr Gly Pro
   1               5                   10                  15
   Tyr Thr Pro Gln Pro Gln Pro Lys Asp
               20                  25

## Description

### BACKGROUND

Forkhead box transcription factors, including forkhead box C1 (FOXC1), are transcription factors characterized by a common 100-amino acid winged-helix DNA-binding domain termed the forkhead box domain, and play important roles in regulating the expression of genes involved in cell growth, survival, differentiation embryonic mesoderm development, migration, and longevity (Nishimura et al., 1998). As a result of the studies described herein, it has been determined that FOXC1 expression in human breast cancer, both at the mRNA and at the protein level, occurs consistently and exclusively in basal-like breast cancers (BLBC). Basal-like breast cancer (BLBC) has a poor prognosis and is often identified by a triple-negative phenotype (ER-/PR-/HER2-) and basal cytokeratins. Recently, however, overexpression of mRNA for the FOXC1 transcription factor has been identified as an important prognostic biomarker of BLBC (Ray et al. 2010). FOXC1 protein expression has also been found to be a significant predictor of overall survival on univariate and multivariate analyses (Ray et al. 2010).

Although many genes have been described to be characteristic biomarkers of certain cancer types, and many others are described to be of functional importance to the survival and maintenance of the malignant phenotype, very few are demonstrated to have robust prognostic significance. This is because very few are critical by themselves and instead are part of extremely large and complex networks of biomolecules whose overall function cannot be determined unless the molecules which are most central and pivotal in the network are identified. FOXC1 has been
demonstrated to have high prognostic significance, being predictive of the high mortality and metastasis rate specifically associated with basal-like breast cancers. Thus, FOXC1 is an indicative and characteristic biomarker of BLBC. The clinical significance of FOXC1 as it relates to basal-like breast cancers is described in detail in International Patent Application No. PCT/US10/44817, filed August 6, 2010.

The clinical significance of FOXC1 expression is not restricted to breast cancer but may extend to other cancers, including but not limited to, brain tumors (such as glioblastoma multiforme, or astrocytoma), colon cancer, gastric cancer, melanoma, neuroendocrine tumors, prostate cancer (e.g., androgen insensitive prostate cancer), renal cell cancer, sarcomas (such as synovial sarcoma), and leukemia. FOXC1 expression has been shown to define biologically and clinically aggressive subsets in such cancers and can be used both as a diagnostic as well as prognostic biomarker for these specific cancer types. Furthermore, FOXC1 is a suitable therapeutic target for BLBC and the other specific cancer types described above. Therefore, development of substances that target FOXC1 and are suitable for use in diagnosis, prognosis and treatment of BLBC and other cancers is desired.

The above described findings have clear and important implications for personalized medicine and personalized cancer care as detection of FOXC1 status of the described specific subsets of patients with breast cancer (and/or other cancers described above) will enable more tailored and specific therapeutic interventions with a greater likelihood of arresting disease progression, extending life expectancy or even achieving a cure.

Ray et al. (Ray, P.S. et al., 2009, Journal of clinical oncology, vol. 27, no. 15, page 11016) disclose that FOXC1 is a dominant determinant of the basal-like phenotype of breast cancer. Taube et al. (Taube, J.H., 2010, PNAS, vol. 107, no. 35, pages 15449-15454) disclose that FOXC1 expression marks basal-like tumors. Muggerud et al. (Muggerud, A.A. et al., 2010, Breast Cancer Research, vol. 12, no. 1, page R3) disclose an increase of methylation frequency of the FOXC1 gene in invasive tumors. Berry et al. (Berry, F.B. et al., 2002, JBC, vol. 227, no. 12, pages 10292-10297) disclose structural domains of FOXC1 including N- and C-terminal activation domains. Ray et al. (Ray, P.S. et al., 2010, Cancer Research, vol. 70, no. 10, pages 3870-3876) disclose that FOXC1 is a potential prognostic biomarker with functional significance in basal-like breast cancer.

### SUMMARY

In a first aspect, the invention is directed to an isolated antibody which binds an antigenic peptide sequence corresponding to the N-terminal transactivation domain of human FOXC1, wherein the antibody is a monoclonal antibody, wherein the antigenic peptide sequence comprises amino acids 51-75 of human FOXC1 (SEQ ID NO:1), and wherein the isolated antibody is used to detect FOXC1 in an in vitro immunoassay.

In various embodiments, the antibody is produced by a hybridoma cell line.

In various embodiments, the antigenic peptide sequence is:
5'-AHAEQYPGGMARAYGPYTPQPQPKD-3' (SEQ ID NO:2); or
5'-C-AHAEQYPGGMARAYGPYTPQPQPKD-3' (SEQ ID NO:3).

In a second aspect, the invention is directed to an *in vitro* method of diagnosing a basal-like breast cancer in a subject having breast cancer or suspected of having breast cancer, the method comprising:
contacting one or more breast cancer cells in a biological sample with a FOXC1 antibody conjugated to a diagnostic agent, wherein the antibody is a monoclonal antibody, and wherein the FOXC1 antibody binds an antigenic peptide sequence corresponding to the N-terminal transactivation domain of human FOXC1 and comprising amino acids 51-75 of human FOXC1 (SEQ ID NO:1);
detecting the presence or absence of FOXC1 in the one or more breast cancer cells; and
diagnosing the subject as having a basal-like breast cancer when FOXC1 is present.

In various embodiments, the biological sample is a primary or metastatic tumor sample, a blood sample, a serum sample, a plasma sample, a lymph sample, a lymph node sample, a cerebrospinal fluid sample, a bone marrow sample, an interstitial fluid sample or a urine sample.

In various embodiments, the diagnostic agent is a radioactive substance, dyes contrast agent, fluorescent compound or molecule, bioluminescent compound or molecule, enzyme or enhancing agent.

In various embosiments, detecting the presence or absence of FOXC1 is accomplished by an in vitro immunoassay, wherein the in vitro immunoassay is optionally selected from immunocytochemistry (ICC), immunohistochemistry (IHC), Western blot or fluorescent in situ hybridization (FISH).

In various embodiments, the method further comprises:
lysing or permeabilizing the one or more breast cancer cells when the in vitro immunoassay uses the contents of whole cells or whole cell preparations.

In various embodiments, the FOXC1 antibody is conjugated to an intracellular delivery agent.

In various embodiments, the antigenic peptide sequence is:
5'-AHAEQYPGGMARAYGPYTPQPQPKD-3' (SEQ ID NO:2); or
5'-C-AHAEQYPGGMARAYGPYTPQPQPKD-3' (SEQ ID NO:3).

The diagnostic agent may be any suitable substance, including a radioactive substance, a dye or contrast agent, a fluorescent compound or molecule, a bioluminescent compound or molecule or an enzyme or enhancing agent.

Detecting the presence or absence of FOXC1 is accomplished by an in vitro immunoassay, such as immunocytochemistry (ICC), immunohistochemistry (IHC), Western blot or fluorescent in situ hybridization (FISH). Alternatively, an in vivo imaging modality may be used, such as magnetic resonance imaging (MRI), positron emission tomography (PET) or microPET, computed tomography (CT), PET/CT combination imager, cooled charged coupled device (CCD), camera optical imaging, optical imaging and single photon emission computed tomography (SPECT). When the presence or absence of FOXC1 is determined by an in vivo method, the FOXC1 antibody or functional fragment thereof should be conjugated to an intracellular delivery agent to facilitate deliver of the antibody or functional fragment thereof to the cytoplasm of target cells.

### BRIEF DESCRIPTION OF THE DRAWINGS

Figure 1 is an immunoblot of MCF-7 human breast cancer cells that were transfected with either FOXC1 (+) or vector (-) using polyclonal commercial FOXC1 antibodies (Santa Cruz Biotechnology) and FOXC1 monoclonal antibodies isolated from hybridoma cell line B2E3. Both antibodies were used at a 1:100 dilution.
Figure 2 are representative images of MCF-7 human breast cancer cells transfected with GFP-FOXC1 (A) wherein immunofluorescence staining was performed using FOXC1 monoclonal antibodies isolated from hybridoma cell line B2E3 to detect successfully transfected cells (B). DAPI was used to stain the DNA of all cells (C).
Figure 3 shows representative images of immunohistochemical staining of FOXC1 on two representative breast cancer samples (FOXC1 positive breast cancer tissue and FOXC1 negative breast cancer tissue) using FOXC1 monoclonal antibodies isolated from hybridoma cell line B2E3.
Figure 4 is the sequence of human forkhead box protein C1 (FOXC1) (SEQ ID NO:1; Accession No. AAH70124). The target antigenic peptide sequence used to generate FOXC1 monoclonal antibodies according to some embodiments is in bold and underlined.

### DETAILED DESCRIPTION

The disclosure is directed to antibodies that specifically bind forkhead box protein C1 (FOXC1) and in vitro diagnostic methods according to the pending claims. According to some embodiments described herein, such anti-FOXC1 antibodies may be used in methods to diagnose and treat basal-like breast cancer and were generated to develop a convenient, pragmatic, and efficient approach to immunohistochemical assays that exploit the diagnostic potential of FOXC1 in breast cancer management.

An antibody as described herein is defined in claim 1 and is a molecule that includes an intact immunoglobulin or one or more portions of an immunoglobulin or immunoglobulin-related molecule that specifically binds to, or is immunologically reactive with an antigenic epitope of a target substance (e.g., a target protein). Such an immunoglobulin or immunoglobulin-related molecule may include monoclonal or polyclonal antibodies of any isotype (e.g., IgA, IgD, IgE, IgG or IgM), modified antibodies or functional antibody fragments.

The term modified antibody includes, but is not limited to genetically engineered or otherwise modified forms of immunoglobulins or functional fragments thereof, such as intrabodies, peptibodies, chimeric antibodies, fully human antibodies, humanized antibodies, bivalent or bispecific antibodies, and conjugate antibodies (e.g., diabodies, triabodies, tetrabodies). The modified antibodies may be monovalent or multivalent. The term functional antibody fragment includes one or more antigen binding fragments of antibodies alone or in combination with other molecules, including, but not limited to Fab', F(ab')₂, Fab, Fv, rlgG, scFv fragments, and single domain fragments. Examples of the types of antibodies, modified antibodies and functional antibody fragments that may be used in accordance with the embodiments described herein are further described in Huson P.J. & Souriau C., Engineered Antibodies, Nature Medicine (2003) 9(1):129-134.

### FOXC1 Antibodies

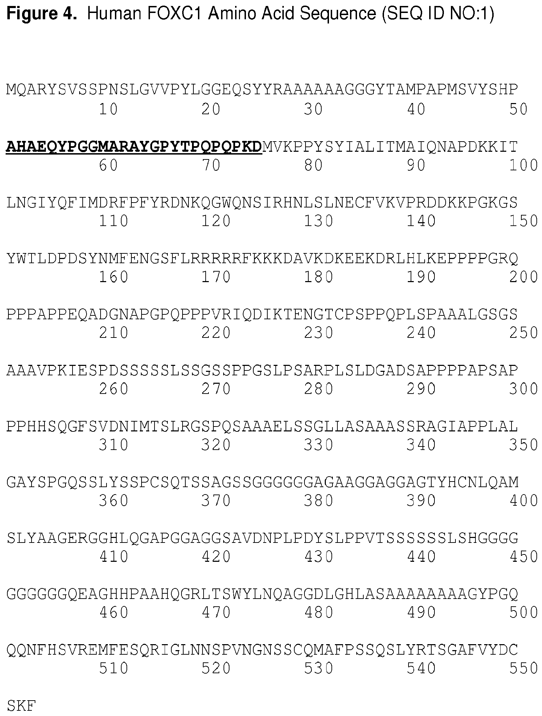

The antibodies described herein specifically bind a target antigenic peptide sequence of human FOXC1 (Figure 4; SEQ ID NO:1). The target antigenic peptide sequence corresponds to an epitope present in the intact, native state of the target protein, FOXC1. The target antigenic peptide sequence corresponds to a continuous epitope (i.e., composed of a contiguous sequence of amino acids in a protein).

Several characteristics are important for selecting a suitable target antigenic peptide sequence within a target protein sequence such as FOXC1. Potential antigenic epitopes within a target protein sequence should have, but are not limited to, the following characteristics: (i) hydrophilic, because most naturally occurring proteins in aqueous solutions have hydrophilic residues on the surface and hydrophobic residues on the interior; (ii) surface-oriented, because antibodies and functional fragments thereof generally bind epitopes on the surface of proteins; and (iii) flexible, because it has been shown that epitopes have a high degree of mobility. Algorithms for predicting protein characteristics such as hydrophilicity/hydrophobicity and secondary structure regions such as alpha-helix, beta sheet and beta turn may aid selection of a potentially exposed, immunogenic internal sequence for antibody generation. A commercial software package may be used to assist with selecting a target antigenic peptide sequence, such as MacVector™, DNAStar™, and PC-Gene™. The antibodies of the inventionbind a target antigenic peptide sequence that corresponds to the N-terminal transactivation domain of FOXC1. The target antigenic peptide sequence is 5'-AHAEQYPGGMARAYGPYTPQPQPKD-3' (SEQ ID NO:2), which corresponds to amino acids 51 to 75 of SEQ ID NO:1 (see Figure 4).

Further described herein is that a target antigenic peptide sequence may be conjugated to a carrier protein to enhance its immunogenicity. The carrier proteins may include epitopes that stimulate T-helper cells, which in turn help induce the B-cell
response against the target antigenic peptide sequence. For example, the carrier peptide may include, but is not limited to, keyhole limpet hemacyanin (KLH), bovine serum albumin (BSA), ovalbumin (OVA) and rabbit serum albumin (RSA).

The conjugation of the target antigenic peptide sequence to the carrier protein may be accomplished by a suitable coupling method. The coupling method may be a carbodiimide method, which uses cabodiimides that can activate the side chain carboxylic groups of aspartic and glutamic acid as well as the carboxyl terminal group to make them reactive sites for coupling with primary amines.Alternatively, gluteraldehyde may be used as a bifunctional coupling reagent that links two compounds through their amino groups.Further, m-Maleimidobenzoyl-N-hydroxysuccinimide ester (MBS) can be used to link peptides to carrier proteins via cysteines. The coupling takes place with the thiol group of cysteine residues. If the chosen sequence does not contain Cys, a Cys residue may be placed at the N- or C-terminus to obtain controlled linking of the peptide to the carrier protein. Thus, the target antigenic peptide sequence described above (SEQ ID NO:2; 5'-AHAEQYPGGMARAYGPYTPQPQPKD-3') may have a cysteine added to the N-terminus to assist in its conjugation to a carrier protein as necessary (e.g., 5'-C-AHAEQYPGGMARAYGPYTPQPQPKD-3'; SEQ ID NO:3).

In some embodiments, the antibody that specifically binds a target antigenic peptide sequence of human FOXC1 (the "FOXC1 antibody" or the "FOXC1 antibodies") is a monoclonal antibody. Monoclonal antibodies are highly specific because they are directed against a single antigenic site. Thus, monoclonal antibodies are useful to improve the selectivity and specificity of diagnostic and analytic assays as well as therapeutic applications as compared to conventional, polyclonal preparations.

Further described herein is that the FOXC1 antibody is a functional fragment of the monoclonal FOXC1 antibody as described above. The functional fragment of the monoclonal FOXC1 antibody may be a portion of the antibody (e.g., a domain), which retains the ability to specifically bind a target antigenic peptide sequence of human FOXC1. Examples of such antibody fragments include: F(ab')₂, Fab', Fab, single-chain Fv (hereinafter, referred to as "scFv"), disulfide-linked Fv (hereinafter, referred to as "dsFv"), a polymer thereof, dimeric V region (hereinafter, referred to as a "diabody"), and peptides or peptibodies containing complementarity determining regions (CDR).

Various methods known within the art may be used for the production of antibodies, including hybridoma, phage library and recombinant techniques (see, e.g., Antibodies: A Laboratory Manual, Harlow E. and Lane D, 1988, Cold Spring Harbor Laboratory Press, Cold Spring Harbor, NY; and Making and Using Antibodies: A Practical Handbook, Howard G and Kaser M, 2006, CRC Press). The FOXC1 antibodies described herein may be generated by a hybridoma cell line. The hybridoma cell line is produced by suitable hybridoma technique known in the art, such as those described by Kohler and Milstein (Kohler & Milstein, 1975). In a hybridoma technique, a host animal (e.g., a mouse or rabbit), is typically immunized with an immunizing agent to elicit lymphocytes that produce or are capable of producing antibodies that will specifically bind to the immunizing agent. Alternatively, the animal may be immunized with cells transfected with a vector containing a nucleic acid molecule encoding FOXC1 or an immunogenic fragment, derivative or variant thereof or the lymphocytes may be immunized in vitro. Such recombinant mAb production methods are described further below.

The immunizing agent may be a target protein antigen (e.g., FOXC1), a fragment thereof or a fusion protein thereof (e.g., the target antigenic peptide sequences of human FOXC1 ; SEQ ID NOs:2-3). The lymphocytes are then fused with an immortalized cell line (e.g., myeloma cells) using a suitable fusing agent, such as polyethylene glycol, to form a hybridoma cell. The hybridoma cells are then cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

The culture medium in which the hybridoma cells are cultured can then be assayed for the presence of monoclonal antibodies directed against the antigen.

Preferably, the binding specificity of monoclonal antibodies produced by the hybridoma cells is determined by a suitable method known in the art, such as immunoprecipitation or an in vitro binding assay, such as radioimmunoassay (RIA) or enzyme-linked immunoabsorbent assay (ELISA).

After the desired hybridoma cells are identified, the clones can be subcloned by limiting dilution procedures and grown by standard methods. (See Coding, Monoclonal Antibodies: Principles and Practice, Academic Press, (1986) pp. 59-103). Suitable culture media for this purpose include, for example, Dulbecco's Modified Eagle's Medium and RPMI-1640 medium. Alternatively, the hybridoma cells may be grown in vivo as ascites in a mammal.

The monoclonal antibodies secreted by the subclones may be isolated or purified from the culture medium or ascites fluid by an immunoglobulin purification procedure known in the art including, but not limited to, protein A-Sepharose, hydroxylapetite chromatography, gel electrophoresis, dialysis, or affinity chromatography.

Antibodies may be made by recombinant DNA methods known in the art. Methods for recombinant production of monoclonal antibodies are described, for example in U.S. Patent No. 4,816,397 to Boss et al. (expired) and U.S. Patent No. 4,816,567 to Cabilly (expired). DNA encoding the FOXC1 monoclonal antibodies described herein can be isolated and sequenced using procedures known in the art (e.g., by using oligonucleotide probes that are capable of binding specifically to genes encoding the heavy and light chains of murine antibodies). Hybridoma cells may serve as a source of such DNA. Once isolated, the DNA can be placed into expression vectors, which are then transfected into host cells (e.g., simian COS cells, Chinese hamster ovary (CHO) cells, or myeloma cells that do not otherwise produce immunoglobulin protein) which synthesize the monoclonal antibodies. The DNA also can be modified, for example, by substituting the coding sequence for human heavy and light chain constant domains in place of the homologous murine sequences or by covalently joining all or part of a coding sequence for a non-immunoglobulin polypeptide to the immunoglobulin coding sequence. Such a non-immunoglobulin polypeptide can be substituted for the constant domains of an antibody of the invention, or can be substituted for the variable domains of one antigen-combining site of an antibody of the invention to create a chimeric bivalent antibody.

Further described herein is that antibody fragments can be prepared by proteolytic hydrolysis of the antibody or by expression in E. coli of DNA encoding the fragment. Antibody fragments can be obtained by pepsin or papain digestion of whole antibodies by conventional methods. For example, antibody fragments can be produced by enzymatic cleavage of antibodies with pepsin to provide a 5S fragment denoted F(ab')₂. This fragment can be further cleaved using a thiol reducing agent, and optionally a blocking group for the sulfhydryl groups resulting from cleavage of disulfide linkages, to produce 3.5S Fab' monovalent fragments. Alternatively, an enzymatic cleavage using pepsin produces two monovalent Fab' fragments and an Fc fragment directly. Other methods of cleaving antibodies, such as separation of heavy chains to form monovalent light-heavy chain fragments, further cleavage of fragments, or other enzymatic, chemical, or genetic techniques may also be used, so long as the fragments bind to the antigen that is recognized by the intact antibody.

In some embodiments, conservative variants of the antibodies may be produced. Such conservative variants employed in antibodies, should retain amino acid residues that are necessary for correct folding and stabilizing between the V_{H} and the V_{L} regions, and will retain the charge characteristics of the residues in order to preserve the low isoelectric point (pi) and low toxicity of the molecules. Amino acid substitutions may be made in the V_{H} and the V_{L} regions to increase yield. Conservative amino acid substitution tables providing functionally similar amino acids are known to one of ordinary skill in the art. Generally, conservative variants will bind the target antigen with an equal to or greater efficiency than the parent monoclonal antibody.

Once the amino acid sequence of an antibody that is specific to a target antigenic peptide sequence of human FOXC1 such as those described above can be determined and a nucleic acid sequence encoding the amino acid sequence can be engineered. In addition, a variety of clones containing functionally equivalent nucleic acid molecules may be constructed, such as nucleic acid molecules which differ in sequence but which encode the same effect or molecule or antibody sequence. Thus, nucleic acids encoding FOXC1 -specific antibodies (such as those specific for the epitope AHAEQYPGGMARAYGPYTPQPQPKD, amino acids 51 to 75 of SEQ ID NO:1), conjugates and fusion proteins are described herein.

### Anti-FOXC1 derivatives and conjugates

In some embodiments, the FOXC1 antibodies may include antibody derivatives that are chemically modified. For example, the antibody derivatives include, but are not limited to, antibodies that have been modified by glycosylation, acetylation, pegylation, phosphorylation, amidation or derivatization by known protecting/blocking groups, proteolytic cleavage, and linkage to a cellular ligand or other protein. Any of numerous chemical modifications may be carried out by known techniques, including, but not limited to, specific chemical cleavage, acetylation, gormylation and metabolic synthesis of tunicamycin. Additionally, the derivative may contain one or more non-natural amino acids.

In other embodiments, the FOXC1 antibody may be conjugated to another substance to form an anti-FOXC1 conjugate. The anti-FOXC1 conjugates described herein can be prepared by known methods of linking antibodies with lipids, carbohydrates, proteins or other atoms and molecules. In one aspect, the anti-FOXC1 conjugate is formed by site-specific conjugation using a suitable linkage or bond. Site-specific conjugation is more likely to preserve the binding activity of an antibody or functional antibody fragment. The substance may be conjugated or attached at the hinge region of a reduced antibody component or antibody fragment via disulfide bond formation. For example, introduction of cysteine residues at the C-terminus of an scFv fragment, such as those introduce in the cys-diabodies described above, allows site-specific thiol-reactive coupling at a site away from the antigen binding site to a wide variety of agents. Alternatively, other linkages or bonds used to form the anti-FOXC1 conjugate may include, but is not limited to, a covalent bond, a noncovalent bond, a sulfide linkage, a hydrazone linkage, a hydrazine linkage, an ester linkage, an amido linkage, and amino linkage, an imino linkage, a thiosemicabazone linkage, a semicarbazone linkage, an oxime linkage and a carbon-carbon linkage.

In one embodiment, the anti-FOXC1 conjugate may include a FOXC1 antibody conjugated to an intracellular delivery agent. In one aspect, the delivery agent is a cell penetrating peptide. Cell penetrating peptides are short peptides that facilitate cellular uptake of various molecules and substances (e.g., small molecules, peptides, proteins and nucleic acids), for delivery of such molecules and substances to the cytoplasm of a cell. This is accomplished by exploiting various cellular processes such as direct plasma membrane penetration, endocytosis mediated transport and translocation through formation of a transitory structure. Cell penetrating peptides that may be used to form an anti-FOXC1 conjugate for intracellular delivery include, but are not limited to, TransActivator of Transcription (TAT) protein (Mie et al. 2003; Wadia et al. 2004; Kameyama et al. 2006), Protein essential during penetration 1 (Pep-1) (Morris et al. 2001). Other delivery agents that may be used to deliver the FOXC1 antibody or functional fragment therein include, but are not limited to, cationic lipids (Weill et al. 2008), poly-L-arginine (Chen & Erlanger 2002); liposomal drugs (Roth et al. 2007) and polymeric carriers (e.g., poly(propyl acrylic acid; PPAA) (Stayton et al. 2005).

In one embodiment, the anti-FOXC1 conjugate may include a FOXC1 antibody conjugated to a diagnostic agent. A "diagnostic agent" is an atom, molecule, compound or other substance that is useful in diagnosing, detecting or visualizing cancer or other conditions associated with FOXC1 by in vitro or ex vivo methods known in the art and described below. According to the embodiments described herein, diagnostic agents may include, but are not limited to, radioactive substances (e.g., radioisotopes, radionuclides, radiolabels or radiotracers), dyes, contrast agents, fluorescent compounds or molecules, bioluminescent compounds or molecules, enzymes and enhancing agents (e.g., paramagnetic ions). In addition, it should be noted that some nanoparticles, for example quantum dots and metal nanoparticles (described below) may also be suitable for use as a detection agent.

Radioactive substances that may be used as diagnostic agents in accordance with the embodiments of the disclosure include, but are not limited to, ¹⁸F, ³²P, ³³P, ⁴⁵Ti, ⁴⁷Sc, ⁵²Fe, ⁵⁹Fe, ⁶²Cu, ⁶⁴Cu, ⁶⁷Cu, ⁶⁷Ga, ⁶⁸Ga, ⁷⁵Sc, ⁷⁷As^{, 86}Y, ⁹⁰Y. ⁸⁹Sr, ⁸⁹Zr, ⁹⁴Tc, ⁹⁴Tc, ^{99m}Tc, ⁹⁹Mo, ¹⁰⁵Pd, ¹⁰⁵Rh, ¹¹¹Ag, ¹¹¹In, ¹²³I, ¹²⁴I, ¹²⁵I, ¹³¹I, ¹⁴²Pr, ¹⁴³Pr, ¹⁴⁹Pm, ¹⁵³Sm, ¹⁵⁴⁻¹⁵⁸¹Gd, ¹⁶¹Tb, ¹⁶⁶Dy, ¹⁶⁶Ho, ¹⁶⁹Er, ¹⁷⁵Lu, ¹⁷⁷Lu, ¹⁸⁶Re, ¹⁸⁸Re, ¹⁸⁹Re, ¹⁹⁴Ir. ¹⁹⁸Au, ¹⁹⁹Au, ²¹¹At, ²¹¹Pb, ²¹²Bi, ²¹²Pb, ²¹³Bi, ²²³Ra and ²²⁵Ac. Paramagnetic ions that may be used as diagnostic agents in accordance with the embodiments of the disclosure include, but are not limited to, ions of transition and lanthanide metals (e.g. metals having atomic numbers of 6 to 9, 21 -29, 42, 43, 44, or 57-71). These metals include ions of Cr, V, Mn, Fe, Co, Ni, Cu, La, Ce, Pr, Nd, Pm, Sm, Eu, Gd, Tb, Dy, Ho, Er, Tm, Yb and Lu.

When the diagnostic agent is a radioactive metal or paramagnetic ion, the agent may be reacted with a reagent having a long tail with one or more chelating groups attached to the long tail for binding these ions. The long tail may be a polymer such as a polylysine, polysaccharide, or other derivatized or derivatizable chain having pendant groups to which may be bound to a chelating group for binding the ions. Examples of chelating groups that may be used according to the disclosure include, but are not limited to, ethylenediaminetetraacetic acid (EDTA), diethylenetriaminepentaacetic acid (DTPA), DOTA, NOTA, NETA, porphyrins, polyamines, crown ethers, bis-thiosemicarbazones, polyoximes, and like groups. The chelate is normally linked to the FOXC1 antibody by a group which enables formation of a bond to the molecule with minimal loss of immunoreactivity and minimal aggregation and/or internal crosslinking. The same chelates, when complexed with non-radioactive metals, such as manganese, iron and gadolinium are useful for MRI, when used along with the antibodies and carriers described herein. Macrocyclic chelates such as NOTA, DOTA, and TETA are of use with a variety of metals and radiometals including, but not limited to, radionuclides of gallium, yttrium and copper, respectively. Other ring-type chelates such as macrocyclic polyethers, which are of interest for stably binding nuclides, such as ²²³Ra for RAIT may be used. In certain embodiments, chelating moieties may be used to attach a PET imaging agent, such as an AI-¹⁸F complex, to a targeting molecule for use in PET analysis.

Bioluminescent and fluorescent compounds or molecules and dyes that may be used as diagnostic agents in accordance with the embodiments of the disclosure include, but are not limited to, fluorescein, fluorescein isothiocyanate (FITC), 5-dimethylamine-1 -napthalenesulfonyl chloride, 4',6-diamidino-2-phenylindole (DAPI), Oregon Green™, rhodamine, Texas red, tetrarhodimine isothiocynate (TRITC), Cy3, Cy5, lanthanide phosphors, green fluorescent protein (GFP), Yellow fluorescent protein (YFP), phycoerythrin and autoquenched fluorescent compounds that are activated by tumor-associated proteases and enzymes (e.g., luciferase, horseradish peroxidase, alkaline phosphatase, nanoparticles, biotin, digoxigenin) or a combination thereof.

Enzymes that may be used as diagnostic agents in accordance with the embodiments of the disclosure include, but are not limited to, horseradish peroxidase, alkaline phosphatase, acid phosphatase, glucose oxidase, β-galactosidase, β-glucoronidase or β-lactamase. Such enzymes may be used in combination with a chromogen, a fluorogenic compound or a luminogenic compound to generate a detectable signal.

Although not falling within the scope of the invention as claimed, further described herein is an anti-FOXC1 conjugate may include a FOXC1 antibody conjugated to a therapeutic agent. A "therapeutic agent" as used herein is an atom, molecule, compound or other substance that is useful in the treatment of cancer or other conditions associated with FOXC1. Examples of therapeutic agents include, but are not limited to, drugs, chemotherapeutic agents, therapeutic antibodies and antibody fragments, toxins, radioisotopes, enzymes (e.g., enzymes to cleave prodrugs to a cytotoxic agent at the site of the tumor), nucleases, hormones, immunomodulators, antisense oligonucleotides, chelators, boron compounds, photoactive agents and dyes.

Chemotherapeutic agents are often cytotoxic or cytostatic in nature and may include alkylating agents, antimetabolites, anti-tumor antibiotics, topoisomerase inhibitors, mitotic inhibitors hormone therapy, targeted therapeutics and immunotherapeutics. The chemotherapeutic agents that may be used as therapeutic agents in accordance with the embodiments of the disclosure include, but are not limited to, 13-cis-Retinoic Acid, 2-Chlorodeoxyadenosine, 5-Azacitidine, 5-Fluorouracil, 6-Mercaptopurine, 6-Thioguanine, actinomycin-D, adriamycin, aldesleukin, alemtuzumab, alitretinoin, all-transretinoic acid, alpha interferon, altretamine, amethopterin, amifostine, anagrelide, anastrozole, arabinosylcytosine, arsenic trioxide, amsacrine, aminocamptothecin, aminoglutethimide, asparaginase, azacytidine, bacillus calmette-guerin (BCG), bendamustine, bevacizumab, bexarotene, bicalutamide, bortezomib, bleomycin, busulfan, calcium leucovorin, citrovorum factor, capecitabine, canertinib, carboplatin, carmustine, cetuximab, chlorambucil, cisplatin, cladribine, cortisone, cyclophosphamide, cytarabine, darbepoetin alfa, dasatinib, daunomycin, decitabine, denileukin diftitox, dexamethasone, dexasone, dexrazoxane, dactinomycin, daunorubicin, decarbazine, docetaxel, doxorubicin, doxifluridine, eniluracil, epirubicin, epoetin alfa, erlotinib, everolimus, exemestane, estramustine, etoposide, filgrastim, fluoxymesterone, fulvestrant, flavopiridol, floxuridine, fludarabine, fluorouracil, flutamide, gefitinib, gemcitabine, gemtuzumab ozogamicin, goserelin, granulocyte - colony stimulating factor, granulocyte macrophage-colony stimulating factor, hexamethylmelamine, hydrocortisone, hydroxyurea, ibritumomab, interferon alpha, interleukin - 2, interleukin-11, isotretinoin, ixabepilone, idarubicin, imatinib mesylate, ifosfamide, irinotecan, lapatinib, lenalidomide, letrozole, leucovorin, leuprolide, liposomal Ara-C, lomustine, mechlorethamine, megestrol, melphalan, mercaptopurine, mesna, methotrexate, methylprednisolone, mitomycin C, mitotane, mitoxantrone, nelarabine, nilutamide, octreotide, oprelvekin, oxaliplatin, paclitaxel, pamidronate, pemetrexed, panitumumab, PEG Interferon, pegaspargase, pegfilgrastim, PEG-L-asparaginase, pentostatin, plicamycin, prednisolone, prednisone, procarbazine, raloxifene, rituximab, romiplostim, ralitrexed, sapacitabine, sargramostim, satraplatin, sorafenib, sunitinib, semustine, streptozocin, tamoxifen, tegafur, tegafur-uracil, temsirolimus, temozolamide, teniposide, thalidomide, thioguanine, thiotepa, topotecan, toremifene, tositumomab, trastuzumab, tretinoin, trimitrexate, alrubicin, vincristine, vinblastine, vindestine, vinorelbine, vorinostat, or zoledronic acid.

Further described herein is that therapeutic antibodies and functional fragments thereof, that may be used as diagnostic agents in accordance with the embodiments of the disclosure include, but are not limited to, alemtuzumab, bevacizumab, cetuximab, edrecolomab, gemtuzumab, ibritumomab tiuxetan, panitumumab, rituximab, tositumomab, and trastuzumab.

Still further described herein is that toxins that may be used as diagnostic agents in accordance with the embodiments of the disclosure include, but are not limited to, ricin, abrin, ribonuclease (RNase), DNase I, Staphylococcal enterotoxin-A, pokeweed antiviral protein, gelonin, diphtheria toxin, Pseudomonas exotoxin, and Pseudomonas endotoxin.

Radioisotopes that may be used as diagnostic agents in accordance with the embodiments of the disclosure include, but are not limited to, ³²P, ⁸⁹Sr, ⁹⁰Y. ^{99m}Tc, "Mo, ¹³¹I, ¹⁵³Sm, ¹⁷⁷Lu, ¹⁸⁶Re, ²¹³Bi, ²²³Ra and ²²⁵Ac.

In another embodiment, the anti-FOXC1 conjugate may include a FOXC1 conjugated to a nanoparticle. The term "nanoparticle" refers to a microscopic particle, the size of which is measured in nanometers, e.g., a particle with at least one dimension less than about 100 nm. Nanoparticles are particularly useful as detectable substances because they are small enough to scatter visible light rather than absorb it. For example, gold nanoparticles possess significant visible light extinction properties and appear deep red to black in solution. As a result, compositions comprising FOXC1 -specific antibody conjugated to nanoparticles can be used for the in vitro imaging of tumors or cancerous cells in a biological sample. At the small end of the size range, nanoparticles are often referred to as clusters. Metal, dielectric, and semiconductor nanoparticles have been formed, as well as hybrid structures (e.g. core-shell nanoparticles). Nanospheres, nanorods, and nanocups are just a few of the shapes that have been grown. Semiconductor quantum dots and nanocrystals are examples of additional types of nanoparticles. Such nanoscale particles, when conjugated to a FOXC1 antibody, can be used as imaging agents for the in vitro detection of tumor cells as described above. Further described herein is that nanoparticles can be used in therapeutic applications as drug carriers that, when conjugated to a PSCA-specific antibody or fragment of the present invention, deliver chemotherapeutic agents, hormonal therapeutic agents, radiotherapeutic agents, toxins, or any other cytotoxic or anti-cancer agent known in the art to cancerous cells that overexpress PSCA on the cell surface.

Further described herein is that any of the anti-FOXC1 conjugates described above may be further conjugated with one or more additional therapeutic agents, diagnostic agents, nanoparticles, carriers or a combination thereof. For example, a FOXC1 antibody or functional FOXC1 antibody fragment may be radiolabeled with ¹³¹I and conjugated to a lipid carrier, such that the anti-FOXC1 -lipid conjugate forms a micelle. The micelle may incorporate one or more therapeutic or diagnostic agents. Alternatively, in addition to
the carrier, the FOXC1 antibody or functional FOXC1 antibody fragment may be conjugated to ¹³¹I (e.g., at a tyrosine residue) and a drug (e.g., at the epsilon amino group of a lysine residue), and the carrier may incorporate an additional therapeutic or diagnostic agent.

### Methods for diagnosing

A FOXC1 antibody such as those described above may be used to target a FOXC1 positive cell, such as cancer cells that express or overexpress FOXC1. FOXC1 expression has been shown to define biologically and clinically aggressive subsets in several types of cancer and may be used both as a diagnostic as well as prognostic biomarker. FOXC1 is also a suitable therapeutic target for cancer, as describe further below. Cancers that are associated with FOXC1 expression may include, but are not limited to, brain tumors (such as glioblastoma multiforme, or astrocytoma), breast cancer, colon cancer, gastric cancer, leukemia, melanoma, neuroendocrine tumors, prostate cancer (e.g., androgen-resistant prostate cancer), renal cell cancer and sarcomas (such as synovial sarcoma).

In particular, FOXC1 expression is an important theranostic biomarker that may be used to clinically define, diagnose prognose and treat primary and metastatic basal-like breast cancer and related basal-like subtypes. The relationship between FOXC1 expression and its role in basal-like breast cancer is described in detail in International Patent Application No. PCT/US10/44817, filed August 6, 2010. Briefly, FOXC1 is elevated only in basal-like molecular subtypes of breast cancers, and has been demonstrated to be of high prognostic significance, as it is predictive of the high mortality and metastasis rate specifically associated with basal-like breast cancers.

Thus, a FOXC1 antibody according to claim 1 is used in methods for diagnosing primary and metastatic basal-like breast cancer according to claim 4. In one embodiment, the FOXC1 antibody is a FOXC1 monoclonal antibody that is produced by a hybridoma cell line.

The FOXC1 antibody described herein may be used to detect cells that express or overexpress FOXC1. This is accomplished by contacting one or more breast cancer cells in a biological sample with the FOXC1 antibody or functional antibody fragment. In some embodiments, the biological sample may by any biological sample that contains or is suspected of containing breast cancer cells including, but not limited to primary or metastatic tumors, blood, serum, plasma, lymph, lymph nodes, cerebrospinal fluid, bone marrow, interstitial fluid, and urine. Further described herein is that the biological sample may be removed from a subject having breast cancer or suspected of having cancer. In the present diagnostic methods, the FOXC1 antibody is conjugated to a diagnostic agent to allow for detecting the presence or absence of FOXC1 in the breast cancer cells. The diagnostic agent may be any suitable radioactive substance, dyes, contrast agent, fluorescent compound or molecule, bioluminescent compound or molecule, enzyme or enhancing agent described in detail above or otherwise known in the art. The diagnostic anti-FOXC1 conjugate may be conjugated to or associated with one or more additional substances described herein, such as carrier lipids or nanoparticles.

The detection of FOXC1 may be by any suitable in vitro method. In one embodiment, an in vitro diagnostic immunoassay will be performed to determine the expression level of FOXC1 in a tissue sample that contains cancer cells (e.g., breast cancer cells). The tissue sample may be taken from a subject having or suspected of having a cancer associated with FOXC1 (e.g. breast cancer) and compared to a normal (i.e., non cancerous) or control tissue sample (i.e., known cancerous or benign tissue sample). In other embodiments, the tissue sample may be assayed for the presence or absence of FOXC1.

Detecting the presence or absence of FOXC1 or detecting an expression level of FOXC1 in a tissue sample is accomplished by any suitable in vitro immunoassay such as immunohistochemistry, immunocytochemistry, fluorescent in situ hybridization (FISH) or other immunofluorescence staining and Western blots. Because plasma membranes are generally not permeable to antibodies, proteins, peptides, drugs and other therapeutic substances, in vitro immunoassays that use the contents of whole cells or whole cell preparations (e.g., immunocytochemistry, immunofluorescence staining, Western blot) should include a step to lyse or permeablize the cells in the tissue sample to allow the FOXC1 antibody or functional antibody fragment to reach its intracellular target, FOXC1.

In another embodiment, the imaging method may include magnetic resonance imaging (MRI), positron emission tomography (PET) or microPET, computed tomography (CT), PET/CT combination imager, cooled charged coupled device (CCD), camera optical imaging, optical imaging (e.g., bioluminescent optical imaging, fluorescent optical imaging, or absorption of reflectance) and single photon emission computed tomography (SPECT).

Cellular plasma membranes are generally not permeable to antibodies, proteins, peptides, drugs and other therapeutic substances. Therefore, for some imaging methods, the diagnostic anti-FOXC1 conjugate must be delivered intracellular. According to some embodiments, the anti-FOXC1 conjugate may be delivered intracellularly by conjugation to or association with an intracellular delivery agent or a carrier. In one embodiment, the delivery agent or carrier may include a cell penetrating peptide or a delivery reagent. Cell penetrating peptides that may be used to form an anti-FOXC1 conjugate for intracellular delivery include, but are not limited to, TAT protein and Pep-1 protein. Other delivery agents that may be used to deliver the FOXC1 antibody or functional fragment therein include, but are not limited to, nanoparticles, cationic lipids, poly-L-arginine; liposomal drugs and polymeric carriers (e.g., poly(propyl acrylic acid) (PPAA). In other embodiments, the anti-FOXC1 conjugate may be delivered intracellular^ by any other suitable method, for example, delivery of genetic material via viral vector, receptor mediated endocytosis and production of intrabodies.

In addition to diagnosing a cancer associated with FOXC1 expression, further described herein is that the anti-FOXC1 conjugate may also be used to stage, and monitor cancer progression according to method that are similar to those described above.

### Methods for treating cancer

Although not falling within the scope of the invention as claimed, further described herein are methods for treating cancer or other conditions associated with overexpression of FOXC1 are provided. Such methods include administering to a subject a therapeutically effective amount of a pharmaceutical composition that includes a FOXC1 antibody, or a functional FOXC1 antibody fragment as described above. The FOXC1 antibody may be a monoclonal antibody, a modified antibody or a functional antibody fragment as described in detail above.

When a FOXC1 antibody is administered as part of a pharmaceutical composition, it must be delivered intracellular^. As described above, intracellular delivery of the FOXC1 antibody may be accomplished by conjugation to or association with an intracellular delivery agent or a carrier or by any other suitable method known in the art. Thus, the FOXC1 antibody or functional fragment may be conjugated to an intracellular delivery agent to allow for delivery of the antibody to the cytoplasm of cells.

"Treating" or "treatment" of a condition may refer to preventing the condition, slowing the onset or rate of development of the condition, reducing the risk of developing the condition, preventing or delaying the development of symptoms associated with the condition, reducing or ending symptoms associated with the condition, generating a complete or partial regression of the condition, or some combination thereof.

A "therapeutically effective amount" or a "therapeutically effective dose" is an amount of a compound that produces a desired therapeutic effect in a subject, such as preventing or treating a target condition or alleviating symptoms associated with the condition. The precise therapeutically effective amount is an amount of the composition that will yield the most effective results in terms of efficacy of treatment in a given subject. This amount will vary depending upon a variety of factors, including but not limited to the characteristics of the therapeutic compound (including activity, pharmacokinetics, pharmacodynamics, and bioavailability), the physiological condition of the subject (including age, sex, disease type and stage, general physical condition, responsiveness to a given dosage, and type of medication), the nature of the pharmaceutically acceptable carrier or carriers in the formulation, and the route of administration. One skilled in the clinical and pharmacological arts will be able to determine a therapeutically effective amount through routine experimentation, namely by monitoring a subject's response to administration of a compound and adjusting the dosage accordingly. For additional guidance, see Remington: The Science and Practice of Pharmacy 21 st Edition, Univ. of Sciences in Philadelphia (USIP), Lippincott Williams & Wilkins, Philadelphia, PA, 2005.

The pharmaceutical composition may include a FOXC1 antibody, modified antibody or a functional antibody fragment by itself or a therapeutic anti-FOXC1 conjugate. The conjugate may include a FOXC1 antibody, modified antibody or a functional antibody fragment conjugated to one or more therapeutic agents as described above. The FOXC1 antibody, or a functional FOXC1 antibody fragment may be a FOXC1 monoclonal antibody is produced by a hybridoma cell line and specifically binds to a target antigenic peptide sequence (e.g., SEQ ID NOs:2-3).

A FOXC1 antibody, modified antibody or a functional antibody fragment or a therapeutic anti-FOXC1 conjugate may be conjugated to or associated with one or more additional substances described herein, such as diagnostic anti-FOXC1 conjugates (described above), unconjugated diagnostic agents, contrast solutions, carrier lipids or nanoparticles.

The pharmaceutical composition may also include a pharmaceutically acceptable carrier. A pharmaceutically acceptable carrier may be a pharmaceutically acceptable material, composition, or vehicle that is involved in carrying or transporting a compound of interest from one tissue, organ, or portion of the body to another tissue, organ, or portion of the body. For example, the carrier may be a liquid or solid filler, diluent, excipient, solvent, or encapsulating material, or some combination thereof. Each component of the carrier must be "pharmaceutically acceptable" in that it must be compatible with the other ingredients of the formulation. It also must be suitable for contact with any tissue, organ, or portion of the body that it may encounter, meaning that it must not carry a risk of toxicity, irritation, allergic response, immunogenicity, or any other complication that excessively outweighs its therapeutic benefits.

The pharmaceutical compositions described herein may be administered by any suitable route of administration. A route of administration may refer to any administration pathway known in the art, including but not limited to aerosol, enteral, nasal, ophthalmic, oral, parenteral, rectal, transdermal (e.g., topical cream or ointment, patch), or vaginal. "Transdermal" administration may be accomplished using a topical cream or ointment or by means of a transdermal patch. "Parenteral" refers to a route of administration that is generally associated with injection, including infraorbital, infusion, intraarterial, intracapsular, intracardiac, intradermal, intramuscular, intraperitoneal, intrapulmonary, intraspinal, intrasternal, intrathecal, intrauterine, intravenous, subarachnoid, subcapsular, subcutaneous, transmucosal, or transtracheal.

### Example 1 : Generation of a FOXC1 Monoclonal Antibody

An anti-FOXC1 antibody was generated as described below.

Antigenic peptide sequence design. An antigenic peptide sequence was designed using the Lasergene software suite by DNASTAR™. The software was used to determine the regions of the FOXC1 protein that maximize hydrophilicity (H), antigenicity (A), and surface probability (SP), but do not contain turns or glycosylation sites. In addition, BLAST searches against gene databases were also performed to ensure that the homology of these peptides with other FOX family genes is low. Based on the results of the design process described above, residues 51 -75 of the human FOXC1 protein were selected as the antigenic peptide sequence as shown below:
5'-AHAEQYPGGMARAYGPYTPQPQPKD-3' (SEQ ID NO:2)

A cysteine residue was added to the N-terminus (shown below) to assist in conjugation to the carrier protein as necessary.
5'-C-AHAEQYPGGMARAYGPYTPQPQPKD-3' (SEQ ID NO:3)

This antigenic peptide sequence is found in the FOXC1 N-terminal transactivation domain. Previous reports have suggested that the FOXC1 N-terminal domain plays an important role in the function of FOXC1 in transcriptional activation (Berry et al., 2002). Therefore, FOXC1 antibodies that specifically bind to this domain are not only useful in diagnostic and prognostic assays, but are likely to be good candidates for treating basal like breast cancers and other cancers that are associated with the expression or overexpression of FOXC because they block the activity of FOXC1.

*Production of hybridoma and antibodies.* Hybridoma and antibody generation was contracted through Pro-Sci Inc. Briefly, Balb/c mice were injected with the FOXC1 peptide antigen sequence described above to generate lymphocytes that produce or are able to produce a complementary monoclonal antibody. Next, immunized mice with high titers of anti-FOXC1 antibody response and positive immunoblot signals were selected by ELISA and immunoblotting,

The hybridoma cell line B2E3 was then generated by fusing myeloma cells with B cells isolated from the spleen of the selected immunized mice. Then ELISA and immunoblotting were used to screen for the positive hybridoma cell clones, followed by two consecutive subclonings. Based on immunoblotting results, several positive clones including the hybridoma cell line B2E3 were expanded in culture. The antibodies produced by the clones were purified using an IgG purification column.

The antibodies produced by the hybridomas as described above may be used for detection of FOXC1 protein and for coupling other molecules to FOXC1 protein. Further, the anti-FOXC1 antibody was tested by immunoblotting, immunofluorescence, and immunohistochemistry as described below.

*Immunoblotting.* MCF-7 human breast cancer cells were transfected with FOXC1 (+) or FOXC1 (-) vectors. Whole cell lysates for western blotting were generated by cell lysis buffer (50 mM Tris-HCl, pH 7.4, 150 mM NaCl, 2 mM EDTA, 1% NP-40, 10% glycerol) supplemented with a protease inhibitor cocktail (Sigma, St Louis, MO). Equal amounts of protein were separated by 10% SDS-PAGE and then transferred onto a nitrocellulose membrane. The remaining steps were conducted according to a standard immunoblotting protocol (Qu *et al*., 2009). Immunoblotting was done with polyclonal commercial FOXC1 antibodies (Santa Cruz Biotechnology), and FOXC1 monoclonal antibodies isolated from hybridoma cell line B2E3. After the primary antibody incubation, the membrane was again washed with PBST three times (5 min each) and then incubated with a horseradish peroxidase (HRP)-linked secondary antibody (Amersham, Piscataway, NJ) at a dilution of 1:4000 in blocking solution. The membrane was washed and bands were visualized using chemiluminescence assays.

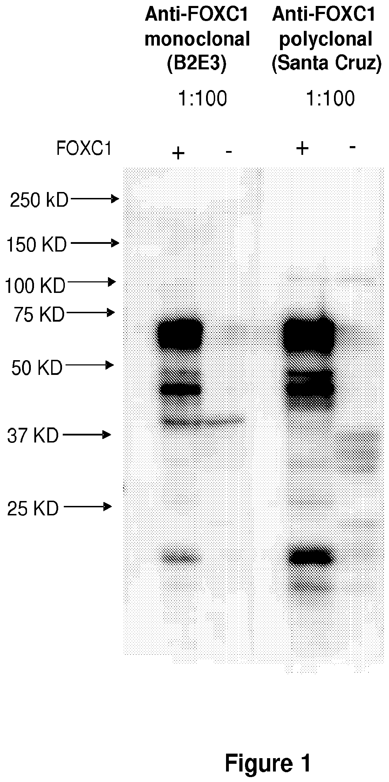

As shown in Figure 1, the FOXC1 monoclonal antibody isolated from hybridoma cell line B2E3 produced fewer non-specific bands as compared to a polyclonal commercial anti-FOXC1 antibody from Santa Cruz Biotechnology Inc. Other available commercial anti-FOXC1 antibodies would produce similar or less desirable results compared to the Santa Cruz anti-FOXC1 antibody in terms of specificity and sensitivity. Therefore, the FOXC1 antibody described herein is more sensitive and is superior as a diagnostic and prognostic tool than antibodies that are currently available.

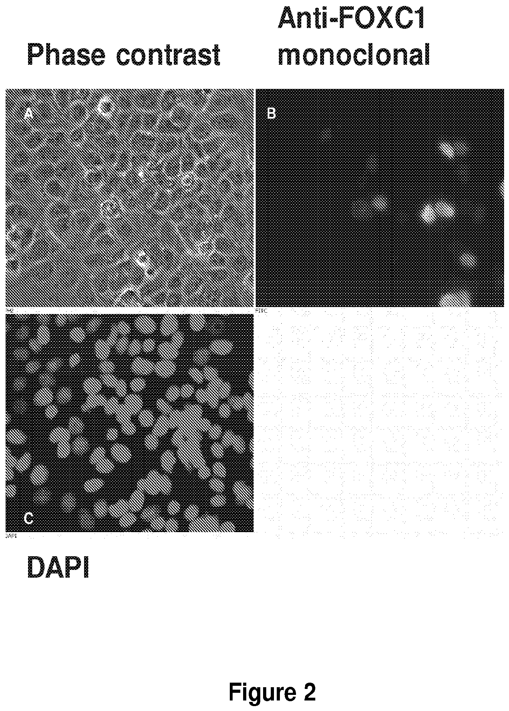

*Immunofluorescence staining.* MCF-7 cells were transiently transfected with GFP-FOXC1 plasmid for 24 h. Then the cells were then digested with trypsin and seeded in chamber slides (BD Biosciences, Franklin Lakes, NJ) (Figure 2A). After 12-h incubation, cells were fixed with 4 % formaldehyde and then permeabilized with PBS containing 0.1 % Triton X-100. Slides were blocked by 5 % BSA for 30 minutes and incubated with a primary FOXC1 monoclonal antibody isolated from hybridoma cell line B2E3 at room temperature for 1 h. Cells were then incubated with an Alexa Fluor 488-conjugated secondary antibody (1:200, Invitrogen) for 30 min (Figure 2B). Slides were washed by PBS three times for 5 minutes each, mounted in DAPI, and observed under a high resolution Nikon TI-E microscope (Figure 2C). The results of this experiment are shown in Figure 2.

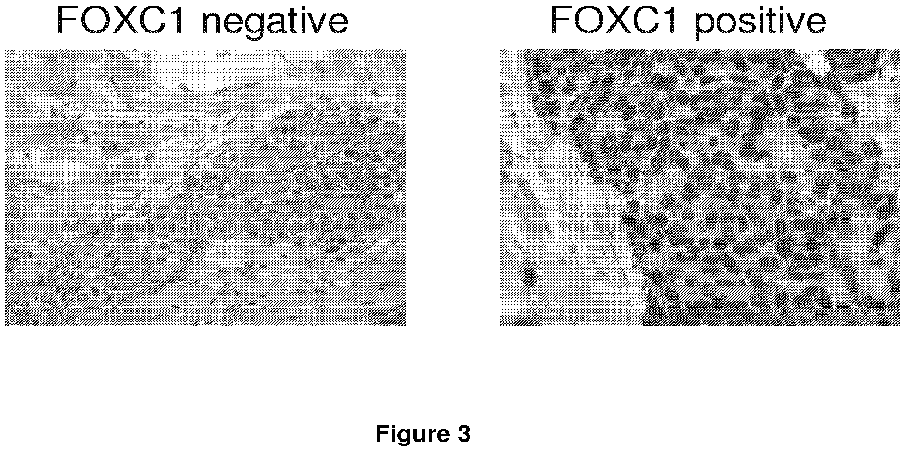

*Immunohistochemistry.* Immunohistochemistry (IHC) was performed according to standard protocols, with the exception of: (1) antigen retrieval was performed for 30 minutes at 100°C in citrate buffer(pH 6.0); (2) primary mouse anti-FOXC1 antibodies were allowed to hybridize for 16 hrs at 4 C (overnight); and (3) the labeling reagent used was a polymer-based anti-mouse antibody (BioCare) with DAB chromogen. The presence of FOXC1 in breast cancer tissues (FOXC1+ and FOXC1-) tissues is shown in Figure 3.

### REFERENCES

Berry FB, Saleem RA, Walter MA (2002). "FOXC1 transcriptional regulation is mediated by N- and C-terminal activation domains and contains a phosphorylated transcriptional inhibitory domain." J. Biol. Chem. 277 (12): 10292-7.
Chen BX, Erlanger BF, Intracellular delivery of monoclonal antibodies, Immunology Letters (2002) 84(1):63-67.
Huson P.J. & Souriau C., Engineered Antibodies, Nature Medicine (2003) 9(1):129-134.
Kameyama S, Horie M, Kikuchi T, Omura T, Takeuchi T, Nakase I, Sugiura Y and Futaki S, Bioconjugate Chem., 2006, 17, 597-602.
Kohler, G. & Milstein, C., Continuous cultures of fused cells secreting antibody of predefined specificity. Nature 256, 495-497 (1975).
Mie M, Takahashi F, Funabashi H, Yanagida Y, Aizawa M and Kobatake E, Intracellular delivery of antibodies using TAT fusion protein A (2003) 310(3):730-734.
Morris MC, Depollier J, Mery J, Heitz F and Divita G, Nat. Biotechnol., 2001 , 19, 1 173-1 176.
Nishimura DY, Swiderski RE, Alward WL, Searby CC, Patil SR, Bennet SR et al (1998). The forkhead transcription factor gene FKHL7 is responsible for glaucoma phenotypes which map to 6p25. Nat Genet 19: 140-7.
Qu Y, Wang J, Sim MS, Liu B, Giuliano A, Barsoum J et al (2009). Elesclomol, counteracted by Akt survival signaling, enhances the apoptotic effect of chemotherapy drugs in breast cancer cells. Breast Cancer Res Treat.
Ray P, Wang J, Qu Y, Sim M, Shamonki J, Bagaria SP et al (2010). FOXC1 Is a Potential Prognostic Biomarker with Functional Significance in Basal-like Breast Cancer. Cancer Research May 15. Ray et al. 2010, Annals of Surgical Oncology, under review.
Ray P, Wang J, Qu Y, Sim M, Shamonki J, Bagaria SP et al (2010). FOXC1 Is a Potential Prognostic Biomarker with Functional Significance in Basal-like Breast Cancer. Cancer Research May 15.
Roth A, Drummond DC, Conrad F, Hayes ME, Kirpotin DB, Benz CC, Marks JD and Liu B, Anti-CD166 single chain antibody-nediated intracellular delivery of liposomal drugs to prostate cancer cells. Molecular Cancer Therapy 2007; 6(10):2737-46.
Stayton PS, EI-Sayed ME, Murthy N, Bulmus V, Lackey C, Cheung C, Hoffman AS, 'Smart' delivery systems for biomolecular therapeutics, Orthod Craniofac Res. 2005; 8(3):219-25.
Wadia JS, Stan RV, Dowdy SF: Transducible TAT-HA fusogenic peptide enhances escape of TAT-fusion proteins after lipid raft macropinocytosis. Nat Med 2004, 10:310-315.
Weill CO, Biri S, Erbacher P, Cationic lipid-mediated intracellular delivery of antibodies into live cells. BioTechniques 44(7) vii-xi (2008).

## Claims

1. An isolated antibody which binds an antigenic peptide sequence corresponding to the N-terminal transactivation domain of human FOXC1, wherein the antibody is a monoclonal antibody, wherein the antigenic peptide sequence comprises amino acids 51-75 of human FOXC1 (SEQ ID NO:1), and wherein the isolated antibody is used to detect FOXC1 in an in vitro immunoassay.

2. The antibody of claim 1, wherein the antibody is produced by a hybridoma cell line.

3. The antibody of any one of claims 1 or 2, wherein the antigenic peptide sequence is:
5'-AHAEQYPGGMARAYGPYTPQPQPKD-3' (SEQ ID NO:2); or
5'-C-AHAEQYPGGMARAYGPYTPQPQPKD-3' (SEQ ID NO:3).

4. An *in vitro* method of diagnosing a basal-like breast cancer in a subject having breast cancer or suspected of having breast cancer, the method comprising:
contacting one or more breast cancer cells in a biological sample with a FOXC1 antibody conjugated to a diagnostic agent, wherein the antibody is a monoclonal antibody, and wherein the FOXC1 antibody binds an antigenic peptide sequence corresponding to the N-terminal transactivation domain of human FOXC1 and comprising amino acids 51-75 of human FOXC1 (SEQ ID NO:1);
detecting the presence or absence of FOXC1 in the one or more breast cancer cells; and
diagnosing the subject as having a basal-like breast cancer when FOXC1 is present.

5. The method of claim 4, wherein the biological sample is a primary or metastatic tumor sample, a blood sample, a serum sample, a plasma sample, a lymph sample, a lymph node sample, a cerebrospinal fluid sample, a bone marrow sample, an interstitial fluid sample or a urine sample.

6. The method of claim 4, wherein the diagnostic agent is a radioactive substance, dyes contrast agent, fluorescent compound or molecule, bioluminescent compound or molecule, enzyme or enhancing agent.

7. The method of claim 4, wherein detecting the presence or absence of FOXC1 is accomplished by an in vitro immunoassay, wherein the in vitro immunoassay is optionally selected from immunocytochemistry (ICC), immunohistochemistry (IHC), Western blot or fluorescent in situ hybridization (FISH).

8. The method of claim 7, further comprising:
lysing or permeabilizing the one or more breast cancer cells when the in vitro immunoassay uses the contents of whole cells or whole cell preparations.

9. The method of claim 4, wherein the FOXC1 antibody is conjugated to an intracellular delivery agent.

10. The method of any one of claims 4-8 wherein the antigenic peptide sequence is:
5'-AHAEQYPGGMARAYGPYTPQPQPKD-3' (SEQ ID NO:2); or
5'-C-AHAEQYPGGMARAYGPYTPQPQPKD-3' (SEQ ID NO:3).

## Patentansprüche

1. Ein isolierter Antikörper, der eine antigene Peptidsequenz entsprechend der N-terminalen Transaktivierungsdomäne von humanem FOXC1 bindet, wobei der Antikörper ein monoklonaler Antikörper ist, wobei die antigene Peptidsequenz die Aminosäuren 51-75 des humanen FOXC1 (SEQ ID NO: 1) umfasst, und wobei der isolierte Antikörper verwendet wird, um FOXC1 in einem *in vitro* Immunoassay zu detektieren.

2. Der Antikörper nach Anspruch 1, wobei der Antikörper durch eine Hybridomazelllinie hergestellt wird.

3. Der Antikörper nach einem der Ansprüche 1 oder 2, wobei die antigene Peptidsequenz ist:
5'-AHAEQYPGGMARAYGPYTPQPQPKD-3' (SEQ ID NO:2); oder
5'-C-AHAEQYPGGMARAYGPYTPQPQPKD-3' (SEQ ID NO:3).

4. Eine *in-vitro*-Methode zur Diagnose eines basalartigen Brustkrebses bei einem Patienten mit Brustkrebs oder Verdacht auf Brustkrebs, wobei das Verfahren umfasst:
Inkontaktbringen einer oder mehrerer Brustkrebszellen in einer biologischen Probe mit einem FOXC1-Antikörper, der an ein diagnostisches Mittel konjugiert ist, wobei der Antikörper ein monoklonaler Antikörper ist und wobei der FOXC1-Antikörper eine antigene Peptidsequenz bindet, die der N-terminalen Transaktivierungsdomäne von humanem FOXC1 entspricht und die Aminosäuren 51-75 des menschlichen FOXC1 (SEQ ID NO: 1) umfasst;
Nachweisen der Anwesenheit oder Abwesenheit von FOXC1 in der einen oder mehreren Brustkrebszellen; und
Diagnostizieren dass der Patient einen basalartigen Brustkrebs hat, wenn FOXC1 vorhanden ist.

5. Das Verfahren nach Anspruch 4, wobei die biologische Probe eine primäre oder metastatische Tumorprobe, eine Blutprobe, eine Serumprobe, eine Plasmaprobe, eine Lymphprobe, eine Lymphknotenprobe, eine Zerebrospinalflüssigkeitsprobe, eine Knochenmarkprobe, eine interstitielle Flüssigkeitsprobe oder eine Urinprobe ist.

6. Das Verfahren nach Anspruch 4, wobei das diagnostische Mittel eine radioaktive Substanz, ein Farbstoffkontrastmittel, eine fluoreszierende Verbindung oder Molekül, eine biolumineszierende Verbindung oder Molekül, ein Enzym oder ein Verstärkungsmittel ist.

7. Das Verfahren nach Anspruch 4, wobei das Nachweisen der Anwesenheit oder Abwesenheit von FOXC1 durch einen *in vitro* Immunoassay erreicht wird, wobei de*r in vitro* Immunoassay gegebenenfalls ausgewählt ist aus Immunozytochemie (ICC), Immunhistochemie (IHC), Western Blot oder Fluoreszenz in situ Hybridisierung (FISCH).

8. Das Verfahren nach Anspruch 7, ferner umfassend:
Lysieren oder Permeabilisieren der einen oder mehrerer Brustkrebszellen, wenn der *in vitro* Immunoassay als Inhalt ganze Zellen oder Ganzzellpräparate verwendet.

9. Das Verfahren nach Anspruch 4, wobei der FOXC1-Antikörper an ein intrazelluläres Abgabemittel konjugiert ist.

10. Verfahren nach einem der Ansprüche 4-8, wobei die antigene Peptidsequenz:
5'-AHAEQYPGGMARAYGPYTPQPQPKD-3' (SEQ ID NO:2); oder
5'-C-AHAEQYPGGMARAYGPYTPQPQPKD-3' (SEQ ID NO:3) ist.

## Revendications

1. Anticorps isolé qui se lie à une séquence de peptide antigénique correspondant au domaine de transactivation N-terminal de la FOXC1 humaine, où l'anticorps est un anticorps monoclonal, où la séquence de peptide antigénique comprend les acides aminés 51-75 de la FOXC1 humaine (SEQ ID NO: 1), et où l'anticorps isolé est utilisé pour détecter FOXC1 dans un dosage immunologique *in vitro.*

2. Anticorps selon la revendication 1, où l'anticorps est produit par une lignée cellulaire d'hybridome.

3. Anticorps selon l'une quelconque des revendications 1 ou 2, où la séquence de peptide antigénique est :
5'-AHAEQYPGGMARAYGPYTPQPQPKD-3' (SEQ ID NO: 2) ; ou
5'-C-AHAEQYPGGMARAYGPYTPQPQPKD-3' (SEQ ID NO: 3).

4. Procédé *in vitro* de diagnostic d'un cancer du sein de type basal chez un sujet présentant un cancer du sein ou suspecté de présenter un cancer du sein, le procédé comprenant :
la mise en contact d'une ou de plusieurs cellules du cancer du sein dans un échantillon biologique avec un anticorps anti-FOXC1 conjugué à un agent diagnostique, où l'anticorps est un anticorps monoclonal, et où l'anticorps anti-FOXC1 se lie à une séquence de peptide antigénique correspondant au domaine de transactivation N-terminal de la FOXC1 humaine et comprenant les acides aminés 51-75 de la FOXC1 humaine (SEQ ID NO: 1) ;
la détection de la présence ou de l'absence de FOXC1 dans les une ou plusieurs cellules du cancer du sein ; et
le diagnostic d'un cancer du sein de type basal chez le sujet lorsque FOXC1 est présente.

5. Procédé selon la revendication 4, dans lequel l'échantillon biologique est un échantillon de tumeur primaire ou métastatique, un échantillon de sang, un échantillon de sérum, un échantillon de plasma, un échantillon de lymphe, un échantillon de ganglion lymphatique, un échantillon de liquide céphalo-rachidien, un échantillon de moelle osseuse, un échantillon de liquide interstitiel ou un échantillon d'urine.

6. Procédé selon la revendication 4, dans lequel l'agent diagnostique est une substance radioactive, un agent de contraste à base de colorants, un composé ou une molécule fluorescent(e), un composé ou une molécule bioluminescent(e), un agent enzymatique ou amplificateur.

7. Procédé selon la revendication 4, dans lequel la détection de la présence ou de l'absence de FOXC1 est réalisée par un dosage immunologique *in vitro,* dans lequel le dosage immunologique *in vitro* est facultativement sélectionné parmi l'immunocytochimie (ICC), l'immunohistochimie (IHC), un transfert de Western ou une hybridation *in situ* en fluorescence (FISH).

8. Procédé selon la revendication 7, comprenant en outre :
la lyse ou la perméabilisation des une ou plusieurs cellules du cancer du sein lorsque le dosage immunologique *in vitro* utilise le contenu de cellules entières ou des préparations de cellules entières.

9. Procédé selon la revendication 4, dans lequel l'anticorps anti-FOXC1 est conjugué à un agent de délivrance intracellulaire.

10. Procédé selon l'une quelconque des revendications 4 à 8, dans lequel la séquence de peptide antigénique est :
5'-AHAEQYPGGMARAYGPYTPQPQPKD-3' (SEQ ID NO: 2) ; ou
5'-C-AHAEQYPGGMARAYGPYTPQPQPKD-3' (SEQ ID NO: 3).
